# EUROPÄISCHE PATENTSCHRIFT

(11) **EP 3 846 873 B1**
(45) Veröffentlichungstag und Bekanntmachung des Hinweises auf die Patenterteilung: **11.12.2024**
(21) Anmeldenummer: 19778792.2
(22) Anmeldetag: 05.09.2019
(51) Int. Cl.: A61M 1/16

(54) **BEHÄLTERANORDNUNG UND VERFAHREN ZUR BEFÜLLUNG DERSELBEN**
CONTAINER SYSTEM AND METHOD TO FILL IT
ENSEMBLE CONTENEUR ET MÉTHODE POUR SON REMPLISSAGE

(30) Priorität: 05.09.2018 DE 102018121675
(43) Veröffentlichungstag der Anmeldung: 14.07.2021
(73) Patentinhaber: Fresenius Medical Care Deutschland GmbH, 61352 Bad Homburg (DE)
(72) Erfinder: BERLICH, Robert, 66606 St. Wendel (DE); MATHIS, Pascal, 66793 Saarwellingen (DE); FU, Gerard, Shanghai 201615 (CN); LASHER, Richard Allen, Knoxville, TN 37932 (US)
(74) Vertreter: Herrmann, Uwe
(86) Internationale Anmeldenummer: PCT/EP2019/000256
(87) Internationale Veröffentlichungsnummer: WO 2020/048628

(56) Entgegenhaltungen:
- EP-B1- 2 696 910
- DE-A1- 19 510 759

## Beschreibung

Die vorliegende Erfindung betrifft eine Behälteranordnung mit einem ersten Behälter, in dem sich ein Konzentrat befindet, das ausgebildet ist, bei der Verdünnung mit einem Lösungsmittel, vorzugsweise mit reinem Wasser, eine gebrauchsfertige Dialyselösung oder einen Bestandteil von dieser zu bilden.

Weiterhin betrifft die vorliegende Erfindung eine derartige Behälteranordnung, bei welcher der erste Behälter leer ist. Beispielsweise ist der erste Behälter in diesem Fall ein Drainagebeutel eines Peritonealdialysesystems, in welchem verbrauchte Dialyselösung aufgefangen wird.

Aus dem Stand der Technik ist es bekannt, für die Dialysebehandlung eines Patienten eine sterile Lösung zu verwenden, die Wasser als Lösungsmittel, einige physiologische Salze, Puffer und Glucose enthält. Der Hauptbestandteil der Lösung ist Wasser, das volumen- und gewichtsmäßig den Hauptteil der Lösung ausmacht. Während des Herstellprozesses, der Lagerung und dem Transport werden somit große Mengen hochreinen Wassers bewegt. Dies hat nicht nur Handhabungs- und Transportnachteile, sondern ist des Weiteren mit dem Nachteil verbunden, dass der Transport und die Lagerung energetische Nachteile mit sich bringen. Aus der DE19510759A1 ist eine Behälteranordnung zur Herstellung von frischer Dialyselösung und/oder Aufnahme von gebrauchter Dialyselösung bekannt, die einen äußeren und einen inneren Behälter aufweist, wobei der innere Behälter ein elastischer Beutel ist, in dem sich ein Konzentrat befindet, das dazu ausgebildet ist, bei der Verdünnung mit Wasser eine gebrauchsfertige Dialyselösung zu bilden, wobei der Beutel so dimensioniert ist, dass er ohne elastische Verformung dem Innenraum des äußeren Behälters entspricht.

Der vorliegenden Erfindung liegt die Aufgabe zugrunde, eine einfach zu handhabende Behälteranordnung zur Herstellung einer Dialyselösung zu schaffen und den Anteil an Wasser, der beim Handling zur Herstellung einer Dialyselösung zu bewegen ist, zu verringern. Zudem ist es eine weitere Aufgabe der vorliegenden Erfindung, einen verbesserten Drainagebeutel für die Peritonealdialyse bereitzustellen.

Diese Aufgabe wird durch eine Behälteranordnung mit den Merkmalen des Anspruchs 1 gelöst.

Danach ist vorgesehen, dass die Behälteranordnung einen zweiten Behälter aufweist, in dem der erste Behälter aufgenommen ist, wobei der erste Behälter eine größere Elastizität aufweist als der zweite Behälter und wobei der erste Behälter ausgebildet ist, sich bis zu dem zweiten Behälter hin auszudehnen. Die vorliegende Erfindung basiert somit auf dem Gedanken, einen ersten Behälter in einem zweiten Behälter anzuordnen, wobei der erste Behälter derart elastisch ausgeführt ist, dass sich dieser bei der Befüllung mit einem Lösungsmittel, insbesondere mit reinem Wasser (Water for injection), oder einer anderen Flüssigkeit bis hin zu dem zweiten Behälter ausdehnen kann. Reines Wasser ist geeignet zur Verwendung in medizinischen Lösungen, insbesondere zur Injektion in den Patienten. Folglich ist reines Wasser üblicherweise sterilisiert.

Der erste Behälter hat somit die Aufgabe, das Konzentrat und die verdünnte vorzugsweise gebrauchsfertige Dialyselösung oder einen Teil von dieser aufzunehmen und der zweite Behälter hat die Aufgabe, der Behälteranordnung die strukturelle Festigkeit zu verleihen und die Ausdehnung des ersten Behälters zu begrenzen.

Durch die vorliegende Erfindung ist es möglich, den Anteil an Wasser zu reduzieren und ein vorzugsweise flüssiges Konzentrat bereitzustellen, welches vorzugsweise adäquat verpackt (stabil, Heißdampf sterilisierbar, ...) ist. Bevorzugt ist es, wenn dieses Konzentrat über einen speziellen Port nur noch durch die Auffüllung mit hochreinem Wasser zur anwendungsfertigen Lösung nahe oder beim Patienten aufgefüllt werden kann.

Vorzugsweise kommt der zweite Behälter nicht mit Flüssigkeit in Kontakt und hat in diesem Fall hinsichtlich der Aufnahme einer Flüssigkeit keine besonderen Eigenschaften zu erfüllen.

Somit ist eine Trennung bzw. Verteilung der Aufgaben (Aufnahme von Flüssigkeit und strukturelle Festigkeit) auf die beiden Behälter möglich, deren Material somit entsprechend ausgewählt werden kann.

Beispielsweise ist es denkbar, dass der zweite Behälter flexibel, aber nicht elastisch ausgebildet ist. Unter "nicht elastisch" ist auch der Fall zu verstehen, dass eine sehr geringe Elastizität vorliegt. Diese ist vorzugsweise so gering, dass der zweite Behälter eine Ausdehnung des ersten Behälters bei dessen Befüllung mit Lösungsmittel begrenzt.

Der erste Beutel ist vorzugsweise flexibel und elastisch ausgeführt. Dabei weist die Folie des ersten Beutels bevorzugt eine Reißdehnung von 400-900% Längs- und Querrichtung auf.

Um eine Isolationswirkung bei der Hitzesterilisation der Behälteranordnung durch ein Luftpolster zu verhindern, ist vorzugsweise vorgesehen, dass sich zwischen dem ersten und dem zweiten Behälter Vakuum befindet. In diesen evakuierten Bereich dehnt sich der erste Behälter bei seiner Befüllung aus, vorzugsweise bis er an dem zweiten Behälter anliegt. Die Luft zwischen den beiden Beuteln kann während der Herstellung sehr einfach durch Schweißen/Verschließen im Vakuum entfernt werden.

Vorzugsweise ist somit vorgesehen, dass der zweite Behälter eine volumenmäßige Begrenzung bei der Ausdehnung des ersten Behälters darstellt und der Behälteranordnung die strukturelle Festigkeit verleiht.

Eine besonders bevorzugte Ausgestaltung ergibt sich, wenn es sich bei dem ersten und/oder bei dem zweiten Behälter um einen Beutel handelt.

Der erste und der zweite Behälter können an einer oder mehreren Stellen unmittelbar oder mittelbar miteinander verbunden sein. Unter einer mittelbaren Verbindung ist zu verstehen, dass der erste und der zweite Behälter an einem Teil der Behälteranordnung fixiert sind, wie beispielsweise an einem Konnektor zur Befüllung und Entleerung des ersten Behälters, aber nicht unmittelbar miteinander in Verbindung stehen.

Bevorzugt ist eine unmittelbare Verbindung der beiden Behälter an einer oder mehreren Stellen bzw. Seiten, z.B. mittels Verschweißung.

Eine bevorzugte Verbindung der Behälter wird somit durch Verschweißen der Behälter erreicht.

Denkbar ist es, dass der erste und der zweite Behälter jeweils ein oberes und ein unteres Ende aufweisen und dass die Behälter an diesen beiden Enden oder an einem dieser Enden miteinander verbunden, insbesondere verschweißt sind. Denkbar sind auch andere Varianten, wie z.B. die Verschweißung der Beutel an nur einer Seite, d.h. an der rechten oder linken Seite.

Weiterhin kann vorgesehen sein, dass der erste Behälter mit einem Konnektor versehen ist, der von dem Inneren des ersten Behälters nach außen führt. Dieser Konnektor bzw. Schlauch mit einem Konnektor dient zur Befüllung des ersten Behälters mit Lösungsmittel bzw. zur Entleerung der gebrauchsfertigen Dialyselösung oder eines Teils von dieser aus dem expandierten ersten Behälter.

Vorzugsweise umgibt der zweite Behälter den ersten Behälter vollständig.

Der zweite Behälter kann die Umverpackung des ersten Behälters darstellen. Eine Funktion der Umverpackung ist eine Wasserdampf-Barriere aufzubauen. Daher besitzt die Folie des zweiten Behälters bevorzugt eine Durchlässigkeit von 0,2-5,0 g/(m²*d) bei 25°C und 40% relativer Feuchte.

Vorzugsweise liegt das Konzentrat in dem ersten Behälter in flüssiger Form vor. Grundsätzlich ist auch die Verwendung eines festen Konzentrats oder die Kombination aus einem festen und einem flüssigen Konzentrat denkbar und von der Erfindung umfasst.

Denkbar ist es weiterhin, dass der der zweite Behälter teilweise oder vollständig um den ersten Behälter gefaltet oder gerollt ist, so dass sich insbesondere bei der Lagerung und beim Transport eine platzsparende Behälteranordnung ergibt.

Die vorliegende Erfindung betrifft ein Verfahren zur Befüllung einer Behälteranordnung gemäß einem der Ansprüche 1 bis 12 mit Lösungsmittel, insbesondere mit reinem Wasser, wobei das Lösungsmittel in den ersten Behälter eingefüllt wird, der sich dabei ausdehnt. Anstatt des Lösungsmittels kann auch eine beliebige andere Flüssigkeit verwendet werden.

Vorzugsweise wird die Befüllung derart vorgenommen, dass sich der erste Behälter bis hin zu dem zweiten Behälter ausdehnt, der eine strukturelle Begrenzung der Ausdehnung des ersten Behälters darstellt.

Eine hinreichende Mischbarkeit der Inhaltsstoffe typischer Dialyselösungen (Glucose, Salze, Puffer) in der notwendigen Konzentration lässt sich mit einer 10-fach (oder höher) konzentrierten Lösung erreichen. Dementsprechend ist das in dem ersten Behälter befindliche Konzentrat vorzugsweise 10-fach oder höher konzentriert.

Vorzugsweise enthält das Konzentrat wenigstens ein Osmotikum und/oder ein oder mehrere Elektrolyte und/oder ein oder mehrere Puffer und besonders bevorzugt abgesehen von dem insgesamt benötigten Wasser alle zur Herstellung einer Dialyselösung erforderlichen Bestandteile.

Es hat sich als in der Praxis weiterhin als vorteilhaft erwiesen, wenn der erste Behälter ein Drainagebeutel eines Peritonealdialysesystems ist.

Vorzugsweise weist bei einer derartigen Anwendung der zweite Behälter eine durchbrochene und / oder netzartige Struktur auf, welche bei vorzugsweise minimalem Materialverbrauch eine zuverlässige Stützstruktur für den ersten Behälter bildet. Beispielsweise kann der zweite Behälter als Netzbeutel und / oder aus Fäden, Streifen oder Kordeln gefertigt sein. Die Verwendung eines derartigen zweiten Behälters ermöglicht es, den ersten Behälter / Drainagebeutel besonders dünnwandig und / oder aus einem besonders elastischen Material zu fertigen, da der zweite Behälter den ersten Behälter stützt und schützt.

An dieser Stelle wird darauf hingewiesen, dass die Begriffe "ein" und "eine" nicht zwingend auf genau eines der Elemente verweisen, wenngleich dies eine mögliche Ausführung darstellt, sondern auch eine Mehrzahl der Elemente bezeichnen können. Ebenso schließt die Verwendung des Plurals auch das Vorhandensein des fraglichen Elementes in der Einzahl ein und umgekehrt umfasst der Singular auch mehrere der fraglichen Elemente.

In anderen Worten ausgedrückt, kann die Erfindung auch wie folgt beschrieben werden:
Aspekt 1 der Erfindung betrifft eine Behälteranordnung mit einem ersten Behälter, in dem sich ein Konzentrat befindet, das ausgebildet ist, bei der Verdünnung mit einem Lösungsmittel, vorzugsweise mit reinem Wasser (Water for injection), eine gebrauchsfertige Dialyselösung oder einen Bestandteil von dieser zu bilden, dadurch gekennzeichnet, dass die Behälteranordnung einen zweiten Behälter aufweist, in dem der erste Behälter aufgenommen ist, wobei der erste Behälter eine größere Elastizität aufweist als der zweite Behälter und wobei der erste Behälter ausgebildet ist, sich bis zu dem zweiten Behälter hin auszudehnen.
Aspekt 2 der Erfindung betrifft eine Behälteranordnung nach Aspekt 1, dadurch gekennzeichnet, dass der zweite Behälter flexibel, aber nicht elastisch ausgebildet ist und/oder dass der erste Beutel flexibel und elastisch ist.
Aspekt 3 der Erfindung betrifft eine Behälteranordnung nach Aspekt 1 oder 2, dass sich zwischen dem ersten und dem zweiten Behälter Vakuum befindet.
Aspekt 4 der Erfindung betrifft eine Behälteranordnung nach einem der vorhergehenden Aspekte, dadurch gekennzeichnet, dass der zweite Behälter eine volumenmäßige Begrenzung bei der Ausdehnung des ersten Behälters darstellt und der Behälteranordnung die strukturelle Festigkeit verleiht.
Aspekt 5 der Erfindung betrifft eine Behälteranordnung nach einem der vorhergehenden Aspekte, dadurch gekennzeichnet, dass es sich bei dem ersten und/oder bei dem zweiten Behälter um einen Beutel handelt.
Aspekt 6 der Erfindung betrifft eine Behälteranordnung nach einem der vorhergehenden Aspekte, dadurch gekennzeichnet, dass der erste und der zweite Behälter an einer oder mehreren Stellen unmittelbar oder mittelbar miteinander verbunden sind.
Aspekt 7 betrifft eine Behälteranordnung nach Aspekt 6, dadurch gekennzeichnet, dass die Verbindung durch Verschweißen erfolgt.
Aspekt 8 der Erfindung betrifft eine Behälteranordnung nach einem der vorhergehenden Aspekte, dadurch gekennzeichnet, dass der erste und der zweite Behälter ein oberes und ein unteres Ende aufweisen und dass die Behälter an diesen beiden Enden oder an einem dieser Enden miteinander verbunden, insbesondere verschweißt sind.
Aspekt 9 der Erfindung betrifft eine Behälteranordnung nach einem der vorhergehenden Aspekte, dadurch gekennzeichnet, dass der erste Behälter mit einem Konnektor versehen ist, der von dem Inneren des ersten Behälters nach außen führt.
Aspekt 10 der Erfindung betrifft eine Behälteranordnung nach einem der vorhergehenden Aspekte dadurch gekennzeichnet, dass der zweite Behälter die Umverpackung des ersten Behälters darstellt.
Aspekt 11 der Erfindung betrifft eine Behälteranordnung nach einem der vorhergehenden Aspekte, dadurch gekennzeichnet, dass das Konzentrat in flüssiger Form vorliegt.
Aspekt 12 der Erfindung betrifft eine Behälteranordnung nach einem der vorhergehenden Aspekte, dadurch gekennzeichnet, dass der der zweite Behälter teilweise oder vollständig um den ersten Behälter gefaltet oder gerollt ist.
Aspekt 13 der vorliegenden Erfindung betrifft ein Verfahren zur Befüllung einer Behälteranordnung gemäß einem der Aspekte 1 bis 12 mit Lösungsmittel, insbesondere mit reinem Wasser (Water for injection), wobei das Lösungsmittel in den ersten Behälter eingefüllt wird, der sich dabei ausdehnt.
Aspekt 14 betrifft ein Verfahren nach Aspekt 13, dadurch gekennzeichnet, dass die Befüllung derart vorgenommen wird, dass sich der erste Behälter bis hin zu dem zweiten Behälter ausdehnt, der eine strukturelle Begrenzung der Ausdehnung des ersten Behälters darstellt.

Weitere Einzelheiten und Vorteile der Erfindung werden anhand eines in der Zeichnung dargestellten Ausführungsbeispiels näher erläutert.

Es zeigen:
- Figur 1:: eine schematische Ansicht einer Behälteranordnung in einer ersten Ausführungsform,
- Figur 2:: Ansichten der Behälteranordnung gemäß Figur 1 in verschiedenen Stadien der Befüllung des ersten Behälters mit Lösungsmittel,
- Figur 3:: eine schematische Ansicht einer Behälteranordnung in einer zweiten Ausführungsform
- Figur 4:: Ansichten der Behälteranordnung gemäß Figur 3 in verschiedenen Stadien der Befüllung des ersten Behälters mit Lösungsmittel.
- Figur 5:: eine schematische Ansicht einer Behälteranordnung in einer dritten Ausführungsform
- Figur 6:: eine Ansicht der Behälteranordnung gemäß Figur 5 bei gefülltem ersten Behälter.

Ein innerer, kleiner und flexibler Beutel, der das Konzentrat aufnimmt, wird von einem weiteren Beutel umschlossen, der größer als der innere flexible Beutel ist und lediglich eine äußere Hülle zur Stabilisierung darstellt. Diese Hülle kann auch gleichzeitig die Sekundärverpackung darstellen.

In diesem Fall kann lediglich am Konnektor noch eine weitere kleinere Schutzverpackung notwendig sein. Zum Herstellen der anwendungsfertigen Lösung dehnt sich der flexible Beutel, d.h. der erste Behälter in den größeren Umbeutel, d.h. in den zweiten Behälter aus. Hierbei ist bevorzugt keine Luft zwischen Innen- und Außenbeutel, d.h. zwischen dem ersten und dem zweiten Behälter. Das Entfernen der Luft ist zu bevorzugen, damit keine Isolationswirkung der eingeschlossenen Luft beim Heißdampfsterilisieren auftritt.

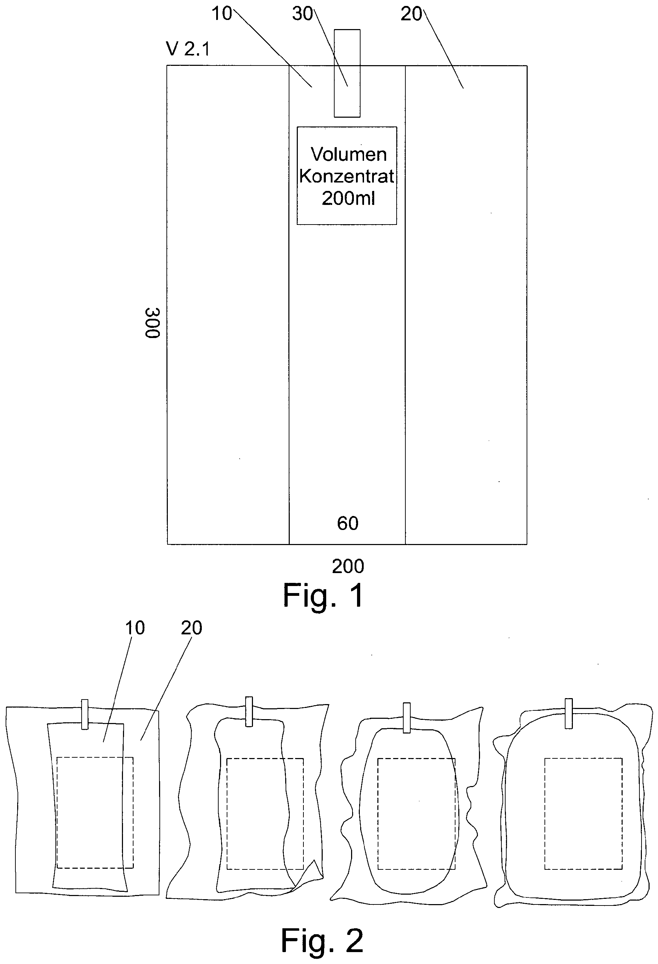

Figur 1 zeigt mit dem Bezugszeichen 10 den ersten Behälter in Form des inneren Beutels und mit dem Bezugszeichen 20 den zweiten Behälter, der ebenfalls als Beutel ausgebildet ist und den äußeren Beutel darstellt, der den inneren Beutel umgibt.

Gemäß Figur 1 sind der innere Beutel 10 und der äußere Beutel 20 am oberen und unteren Ende miteinander verschweißt. Die Beutelausdehnung des inneren Beutels erfolgt hierbei kontrolliert in beide Seiten des äußeren Beutels bis zur vollständigen Ausfüllung, wie dies aus Figur 2 hervorgeht, die die Befüllung zu verschiedenen Zeitpunkten darstellt.

Der innere Beutel 10 ist mit einem Konnektor 30 versehen, mittels dessen Lösungsmittel bzw. Wasser in den inneren Beutel 10 eingefüllt und die gebrauchsfertige Lösung aus dem inneren Beutel 10 abgeführt werden kann.

In dem dargestellten Ausführungsbeispiel weist der erste Behälter 10, d.h. der innere Beutel ein Volumen von 200 ml auf und erstreckt sich über die gesamte Höhe des zweiten Beutels, der die Umverpackung darstellt. Diese Höhe beträgt in dem Ausführungsbeispiel 300 mm. Die Breite des ersten Beutels 10 beträgt 60 mm und die Breite des zweiten Beutels 20 beträgt 200 mm.

Alle der vorgenannten Maße sind nicht beschränkend, sondern nur exemplarisch und beziehen sich auf die Behälteranordnung vor der Befüllung des ersten Beutels 10 mit Lösungsmittel, d.h. im nicht ausgedehnten Zustand.

Durch die Befüllung mit Lösungsmittel, das vorzugsweise durch reines Wasser gebildet wird, wird eine Beutelanordnung erhalten, die ein Volumen von 2 l an gebrauchsfertiger Lösung aufnimmt.

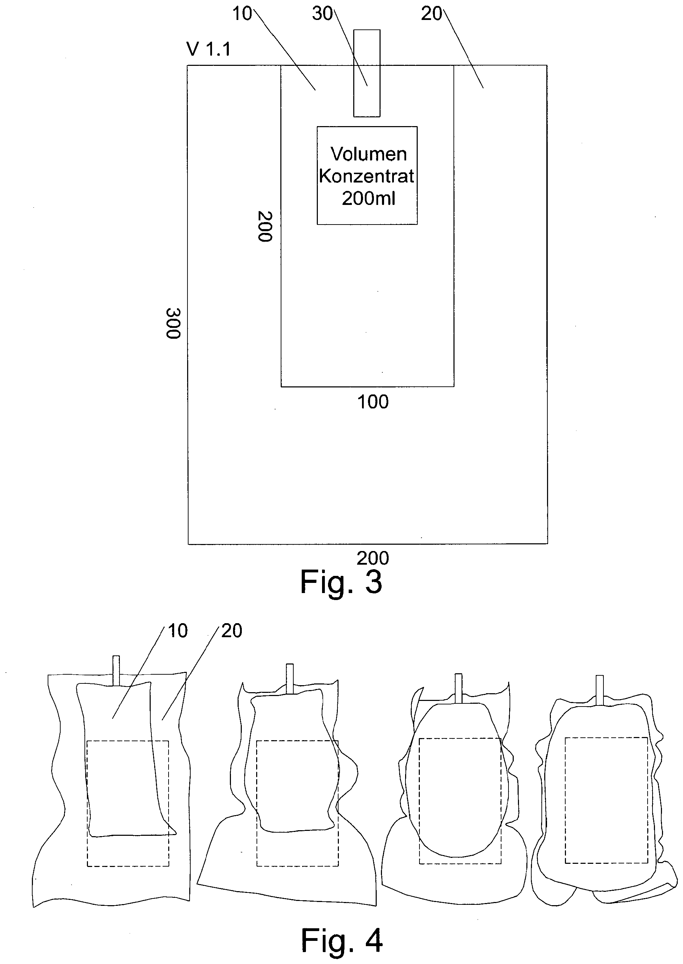

In einer weiteren Variante gemäß Figur 3 ist der innere, das Flüssigkonzentrat enthaltende Beutel 10 nur an dessen Oberseite (Schlaucheinschweißung) mit dem äußeren Beutel 20 verbunden. Hierdurch muss zwar in einem vorgelagerten Prozessschritt der innere Beutel 10 vorverschweißt werden; jedoch kann eine Verschweißung mit dem äußeren Beutel 20 weggelassen werden, was hinsichtlich Produktions-Technologie Vorteile hat.

In dem dargestellten Ausführungsbeispiel gemäß Figur 3 weist der erste Behälter 10, d.h. der innere Beutel ein Volumen von 200 ml auf und erstreckt sich nicht über die gesamte Höhe des zweiten Beutels, der die Umverpackung darstellt. Die Höhe des ersten Behälters 10 beträgt in dem Ausführungsbeispiel nach Figur 3 200 mm und die des zweiten Behälters 20 300 mm. Die Breite des ersten Beutels 10 beträgt 100 mm und die Breite des zweiten Beutels 20 beträgt 200 mm.

Auch für dieses Ausführungsbeispiel gilt, dass alle der vorgenannten Maße nicht beschränkend, sondern nur exemplarisch sind und sich auf die Behälteranordnung vor der Befüllung des ersten Beutels 10 mit Lösungsmittel, d.h. im nicht ausgedehnten Zustand beziehen.

Durch die Befüllung mit Lösungsmittel, das vorzugsweise durch reines Wasser gebildet wird, wird eine Beutelanordnung erhalten, die ein Volumen von 2 I an gebrauchsfertiger Lösung aufnimmt.

Wie dies aus Figur 4 hervorgeht, dehnt sich der erste Beutel 10 bei dessen Befüllung mit reinem Wasser zu beiden Seiten und nach unten zu dem zweiten Beutel 20 hin aus.

Grundsätzlich kann die Dimensionierung von beiden Beuteln und Varianten derart vorgenommen werden, dass verschiedene Endvolumina realisiert werden können.

Die Abmaße und Verhältnisse der Maße der beiden Beutel richten sich nach Endvolumen und Flexibilität des Innenbeutels bzw. des ersten Behälters.

In einer bevorzugten Ausführungsform liegt das Ausgangsvolumen bei 0,1 Liter - 6,5 Liter, bevorzugt 0,2 Liter - 2 Liter, besonders bevorzugt 0,3 Liter - 1 Liter, und das Endvolumen bei 1 Liter - 65 Liter, bevorzugt 1,5 Liter - 12 Liter, besonders bevorzugt 2 Liter - 5 Liter.

Die leeren überstehenden Teile des Außenbeutels, d.h. des zweiten Behälters können für den Transport und Lagerung um den Beutel gefaltet, gerollt werden. Es ist vorzugsweise sichergestellt, dass ein einfaches Entfalten während des Füllens durch Expansion des Innenbeutels erfolgt.

Die wesentliche Idee des flexiblen Innenbeutels 10, der geschützt in einem äußeren Beutel 20 transportiert und später beim Füllen in diesen expandieren kann, besteht darin, dass die für die Funktion notwendige Flexibilität und Umverpackung/Stützwirkung getrennt ist. Dadurch kann eine kostengünstigere Materialauswahl erfolgen. Der flexible Innenbeutel mit hohem Anteil an teuren Materialien ist flächenmäßig deutlich kleiner und hat damit einen deutlich geringeren Verbrauch, als der äußere größere Beutel mit weniger anspruchsvollen und günstigeren Materialien.

In einer bevorzugten Ausführungsform sind als Vorteile der vorliegenden Erfindung zu nennen:
Deutlich weniger Umverpackung bzw. weniger Umverpackung durch Integration einer Stützfunktion und Schutzfunktion in einer Folie, dadurch weniger Abfall und Kosteneinsparungen.

Weniger Lager- und Transportvolumen und geringeres Gewicht. Dadurch lässt sich eine bessere Ausfüllung von Tertiärverpackungen erreichen und es bedarf weniger Tertiärverpackung, d.h. Karton pro Lösungsbeutel.

Es wird nur ein kleinerer Lösungsbeutel 10 benötigt. Somit ergibt sich ein kleineres Oberflächen-Nolumenverhältnis, das einen geringen Wasserverlust begünstigt und der Vorteil, dass der befüllte Lösungsbeutel überfüllt werden kann, weil keine oder nur kürzere Transportanforderungen zu erfüllen sind oder entsprechende steife Stützgebilde den Beutel schützen.

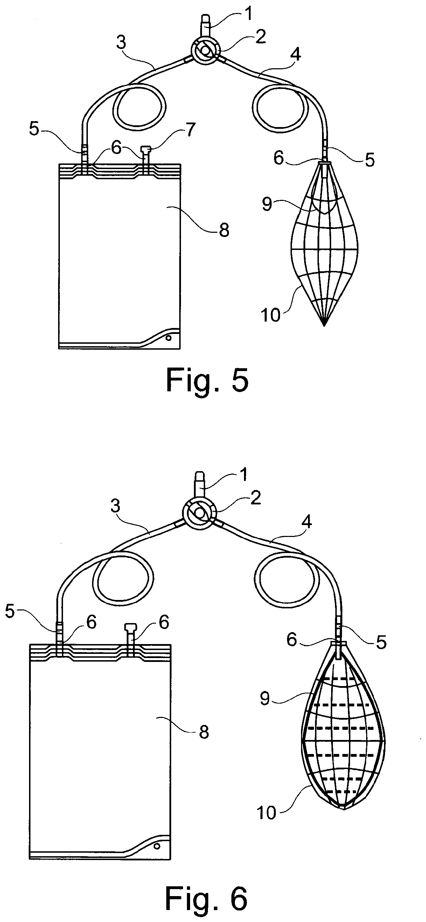

Bei der Ausführungsform aus Fig. 5 wird die Erfindung auf ein Peritonealdialysesystem mit einem Drainagebeutel 9 als erstem Behälter angewandt. Der Drainagebeutel 9 ist in einem zweiten Behälter 10, welcher den Drainagebeutel 9 netzartig / nach Art eines Netzbeutels umgibt, aufgenommen. Drainageflüssigkeit / verbrauchte Dialyseflüssigkeit kann von einem Patientenkonnektor 2 nach Entfernung der Kappe 1 und Anschluss an den Patienten über eine Leitung 4, einen Verbindungskonnektor 5 und einen Verbindungsschlauch 6 in den Drainagebeutel 9 fließen.

Über eine Leitung 3 kann frische Dialyseflüssigkeit von einem Behälter 8 dem Patienten zugeführt werden.

Wie in Fig. 6 gezeigt, dehnt sich der Drainagebeutel 9 innerhalb des zweiten Behälters 10 aus, wenn Flüssigkeit, insbesondere verbrauchte Dialyseflüssigkeit in den Drainagebeutel 9 eingeleitet wird.

## Patentansprüche

1. Behälteranordnung mit einem ersten Behälter (10), wobei es sich bei dem ersten Behälter (10) entweder um einen Behälter, in dem sich ein Konzentrat befindet, das dazu ausgebildet ist, bei der Verdünnung mit einem Lösungsmittel, vorzugsweise mit reinem Wasser (Water for injection), eine gebrauchsfertige Dialyselösung oder einen Bestandteil von dieser zu bilden, oder um einen leeren als Drainagebeutel für die Dialyse geeigneten Behälter handelt, **dadurch gekennzeichnet, dass** die Behälteranordnung einen zweiten Behälter (20) aufweist, in dem der erste Behälter (10) aufgenommen ist, wobei der erste Behälter (10) eine größere Elastizität aufweist als der zweite Behälter (20) und wobei der erste Behälter (10) ausgebildet ist, sich bis zu dem zweiten Behälter (20) hin auszudehnen.

2. Behälteranordnung nach Anspruch 1, **dadurch gekennzeichnet, dass** der zweite Behälter (20) flexibel, aber nicht elastisch ausgebildet ist und/oder dass der erste Beutel (10) flexibel und elastisch ist.

3. Behälteranordnung nach Anspruch 1 oder 2, dass sich zwischen dem ersten und dem zweiten Behälter (10,2 0) Vakuum befindet.

4. Behälteranordnung nach einem der vorhergehenden Ansprüche, **dadurch gekennzeichnet, dass** der zweite Behälter (20) eine volumenmäßige Begrenzung bei der Ausdehnung des ersten Behälters (10) darstellt und der Behälteranordnung die strukturelle Festigkeit verleiht.

5. Behälteranordnung nach einem der vorhergehenden Ansprüche, **dadurch gekennzeichnet, dass** es sich bei dem ersten und/oder bei dem zweiten Behälter (10, 20) um einen Beutel handelt.

6. Behälteranordnung nach einem der vorhergehenden Ansprüche, **dadurch gekennzeichnet, dass** der erste und der zweite Behälter (10,20) an einer oder mehreren Stellen unmittelbar oder mittelbar miteinander verbunden sind.

7. Behälteranordnung nach Anspruch 6, **dadurch gekennzeichnet, dass** die Verbindung durch Verschweißen erfolgt.

8. Behälteranordnung nach einem der vorhergehenden Ansprüche, **dadurch gekennzeichnet, dass** der erste und der zweite Behälter (10, 20) ein oberes und ein unteres Ende aufweisen und dass die Behälter an diesen beiden Enden oder an einem dieser Enden miteinander verbunden, insbesondere verschweißt sind.

9. Behälteranordnung nach einem der vorhergehenden Ansprüche, **dadurch gekennzeichnet, dass** der erste Behälter (10) mit einem Konnektor (5) versehen ist, der von dem Inneren des ersten Behälters nach außen führt.

10. Behälteranordnung nach einem der vorhergehenden Ansprüche, **dadurch gekennzeichnet, dass** der zweite Behälter (20) die Umverpackung des ersten Behälters (10) darstellt.

11. Behälteranordnung nach einem der vorhergehenden Ansprüche, **dadurch gekennzeichnet, dass** es sich bei dem ersten Behälter (10) um einen Behälter handelt, in dem sich ein Konzentrat befindet, das ausgebildet ist, bei der Verdünnung mit einem Lösungsmittel, vorzugsweise mit reinem Wasser (Water for injection), eine gebrauchsfertige Dialyselösung oder einen Bestandteil von dieser zu bilden, und das Konzentrat in flüssiger Form vorliegt.

12. Behälteranordnung nach einem der vorhergehenden Ansprüche, **dadurch gekennzeichnet, dass** der der zweite Behälter (20) teilweise oder vollständig um den ersten Behälter (10) gefaltet oder gerollt ist.

13. Behälteranordnung nach einem der vorhergehenden Ansprüche, **dadurch gekennzeichnet, dass** der erste Behälter (10) ein Drainagebeutel eines Peritonealdialysesystems ist.

14. Behälteranordnung nach Anspruch 13, **dadurch gekennzeichnet, dass** der zweite Behälter (20) eine durchbrochene und / oder netzartige Struktur aufweist.

15. Verfahren zur Befüllung einer Behälteranordnung gemäß einem der Ansprüche 1 bis 14 mit Flüssigkeit oder Lösungsmittel, insbesondere mit reinem Wasser (Water for injection), wobei die Flüssigkeit oder das Lösungsmittel in den ersten Behälter (10) eingefüllt wird, der sich dabei ausdehnt.

16. Verfahren nach Anspruch 15, **dadurch gekennzeichnet, dass** die Befüllung derart vorgenommen wird, dass sich der erste Behälter (10) bis hin zu dem zweiten Behälter (20) ausdehnt, der eine strukturelle Begrenzung der Ausdehnung des ersten Behälters (10) darstellt.

## Claims

1. Container arrangement having a first container (10), wherein the first container (10) is either a container in which a concentrate is located that is configured to form a ready-to-use dialysis solution or a component thereof on a dilution with a solvent, preferably with pure water (water for injection), or an empty container suitable as a drainage bag for dialysis, **characterized in that** the container arrangement has a second container (20) in which the first container (10) is received, with the first container (10) having a greater elasticity than the second container (20) and with the first container (10) being configured to expand up to the second container (20).

2. Container arrangement in accordance with claim 1, **characterized in that** the second container (20) is flexible, but not elastic; and/or **in that** the first bag (10) is flexible and elastic.

3. Container arrangement in accordance with claim 1 or claim 2, **characterized in that** there is a vacuum (10, 20) between the first and second containers.

4. Container arrangement in accordance with one of the preceding claims, **characterized in that** the second container (20) represents a boundary by volume on the expansion of the first container (10) and gives the container arrangement structural strength.

5. Container arrangement in accordance with one of the preceding claims, **characterized in that** the first and/or second containers (10, 20) is/are a bag/bags.

6. Container arrangement in accordance with one of the preceding claims, **characterized in that** the first and second containers (10, 20) are directly or indirectly connected to one another at one or more points.

7. Container arrangement in accordance with claim 6, **characterized in that** the connection takes place by welding.

8. Container arrangement in accordance with one of the preceding claims, **characterized in that** the first and second containers (10, 20) have an upper end and a lower end; and **in that** the containers are connected, in particular welded, to one another at these two ends or at one of these ends.

9. Container arrangement in accordance with one of the preceding claims, **characterized in that** the first container (10) is provided with a connector (5) that leads from the interior of the first container to the exterior.

10. Container arrangement in accordance with one of the preceding claims, **characterized in that** the second container (20) represents the outer packaging of the first container (10).

11. Container arrangement in accordance with one of the preceding claims, **characterized in that** first container (10) is a container in which a concentrate is located that is configured to form a ready-to-use dialysis solution or a component thereof on a dilution with a solvent, preferably with pure water (water for injection), and the concentrate is present in liquid form.

12. Container arrangement in accordance with one of the preceding claims, **characterized in that** the second container (20) is partly or fully folded or rolled around the first container (10).

13. Container arrangement in accordance with one of the preceding claims, **characterized in that** the first container (10) is a drainage bag of a peritoneal dialysis system.

14. Container arrangement in accordance with claim 13, **characterized in that** the second container (20) has a perforated and / or net-like structure.

15. Method of filling a container arrangement in accordance with one of the claims 1 to 14 with liquid or solvent, in particular with pure water (water for injection), wherein the liquid or solvent is filled into the first container that expands in this process.

16. Method in accordance with claim 15, **characterized in that** the filling is carried out such that the first container (10) expands up to the second container (20) that represents a structural boundary of the expansion of the first container (10).

## Revendications

1. Agencement de récipients avec un premier récipient (10), le premier récipient (10) étant soit un récipient dans lequel se trouve un concentré configuré pour former une solution de dialyse prête à l'emploi ou un constituant de celle-ci lors de la dilution avec un solvant, de préférence avec de l'eau pure (eau pour injection), soit un récipient vide convenant comme poche de drainage pour la dialyse, **caractérisé en ce que** l'agencement de récipients présente un deuxième récipient (20) dans lequel est logé le premier récipient (10), le premier récipient (10) présentant une élasticité supérieure à celle du deuxième récipient (20) et le premier récipient (10) étant configuré pour se dilater jusqu'au deuxième récipient (20).

2. Agencement de récipients selon la revendication 1, **caractérisé en ce que** le deuxième récipient (20) est configuré sous forme flexible mais non élastique et/ou **en ce que** la première poche (10) est flexible et élastique.

3. Agencement de récipients selon la revendication 1 ou 2, **caractérisé en ce que** du vide se trouve entre le premier et le deuxième récipient (10, 20).

4. Agencement de récipients selon l'une quelconque des revendications précédentes, **caractérisé en ce que** le deuxième récipient (20) constitue une limite volumétrique à la dilatation du premier récipient (10) et confère la résistance structurelle à l'agencement de récipients.

5. Agencement de récipients selon l'une quelconque des revendications précédentes, **caractérisé en ce que** le premier et/ou le deuxième récipient (10, 20) sont une poche.

6. Agencement de récipients selon l'une quelconque des revendications précédentes, **caractérisé en ce que** le premier et le deuxième récipients (10, 20) sont reliés directement ou indirectement entre eux en un ou plusieurs emplacements.

7. Agencement de récipients selon la revendication 6, **caractérisé en ce que** la liaison est effectuée par soudage.

8. Agencement de récipients selon l'une quelconque des revendications précédentes, **caractérisé en ce que** le premier et le deuxième récipients (10, 20) présentent une extrémité supérieure et une extrémité inférieure et **en ce que** les récipients sont reliés entre eux, notamment soudés, à ces deux extrémités ou à l'une de ces extrémités.

9. Agencement de récipients selon l'une quelconque des revendications précédentes, **caractérisé en ce que** le premier récipient (10) est muni d'un connecteur (5) menant de l'intérieur du premier récipient vers l'extérieur.

10. Agencement de récipients selon l'une quelconque des revendications précédentes, **caractérisé en ce que** le deuxième récipient (20) constitue le suremballage du premier récipient (10).

11. Agencement de récipients selon l'une quelconque des revendications précédentes, **caractérisé en ce que** le premier récipient (10) consiste en un récipient dans lequel se trouve un concentré configuré pour former une solution de dialyse prête à l'emploi ou un constituant de celle-ci lors de la dilution avec un solvant, de préférence avec de l'eau pure (eau pour injection), et le concentré se présente sous forme liquide.

12. Agencement de récipients selon l'une quelconque des revendications précédentes, **caractérisé en ce que** le deuxième récipient (20) est partiellement ou totalement plié ou enroulé autour du premier récipient (10).

13. Agencement de récipients selon l'une quelconque des revendications précédentes, **caractérisé en ce que** le premier récipient (10) est une poche de drainage d'un système de dialyse péritonéale.

14. Agencement de récipients selon la revendication 13, **caractérisé en ce que** le deuxième récipient (20) présente une structure ajourée et/ou réticulée.

15. Procédé de remplissage d'un agencement de récipients selon l'une quelconque des revendications 1 à 14 avec un liquide ou un solvant, notamment de l'eau pure (eau pour injection), le liquide ou le solvant étant versé dans le premier récipient (10) qui se dilate alors.

16. Procédé selon la revendication 15, **caractérisé en ce que** le remplissage est effectué de telle sorte que le premier récipient (10) se dilate jusqu'au deuxième récipient (20), qui constitue une délimitation structurelle de la dilatation du premier récipient (10).
